Europäisches Patentamt

European Patent Office

Office européen des brevets

(19)

(11)    **EP 0 598 805 B1**

(12)            **EUROPÄISCHE PATENTSCHRIFT**

(45) Veröffentlichungstag und Bekanntmachung des Hinweises auf die Patenterteilung:
**25.02.1998  Patentblatt 1998/09**

(21) Anmeldenummer: **92917619.6**

(22) Anmeldetag: **12.08.1992**

(51) Int. Cl.$^6$: **A61B 17/39**

(86) Internationale Anmeldenummer:
**PCT/DE92/00675**

(87) Internationale Veröffentlichungsnummer:
**WO 93/03680 (04.03.1993  Gazette 1993/06)**

(54) **ANORDNUNG ZUM SCHNEIDEN VON BIOLOGISCHEM GEWEBE MIT HOCHFREQUENZSTROM**

ARRANGEMENT FOR CUTTING BIOLOGICAL TISSUES WITH HIGH-FREQUENCY CURRENT

DISPOSITIF D'INCISION DE TISSUS BIOLOGIQUES AVEC UN COURANT HAUTE FREQUENCE

(84) Benannte Vertragsstaaten:
**DE FR GB IT**

(30) Priorität: **12.08.1991 DE 4126608**

(43) Veröffentlichungstag der Anmeldung:
**01.06.1994  Patentblatt 1994/22**

(73) Patentinhaber:
**Karl Storz GmbH & Co.
D-78532 Tuttlingen (DE)**

(72) Erfinder:
- **LINDENMEIER, Heinz
D-8033 Planegg (DE)**
- **LOHR, Georg
D-8012 Ottobrunn (DE)**
- **FASTENMEIER, Karl
D-8000 München 83 (DE)**

- **FLACHENECKER, Gerhard
verstorben (DE)**

(74) Vertreter:
**Münich, Wilhelm, Dr. et al
Kanzlei Münich, Steinmann, Schiller
Wilhelm-Mayr-Str. 11
80689 München (DE)**

(56) Entgegenhaltungen:
**EP-A- 0 219 568**

- **EP-A 0 316 469**
- **u. Tietze, Ch. Schenk, "Halbleiter -
Schaltungstechnik", fünfte Auflage, Springer
Verlag Berlin, Heidelberg, New York 1980, Seiten
698 - 701**

## Beschreibung

## Technisches Gebiet

Die Erfindung bezieht sich auf eine Anordnung zum Schneiden von biologischem Gewebe mit Hochfrequenzstrom gemäß dem Oberbegriff des Patentanspruchs 1.

## Stand der Technik

Hochfrequenzströme werden in der Chirurgie zum Schneiden von biologischem Gewebe oder zum Koagulieren, d. h. Blutstillen verwendet. Beim Schneiden wird eine nahezu kontinuierliche Hochfrequenzleistung zugeführt. Ein Problem der Hochfrequenzchirurgie ist dabei die richtige Leistungsdosierung beim Schneiden. Bei einer zu niedrig eingestellten Leistung wird das Gewebe mechanisch stark belastet, es kann nicht zügig geschnitten werden oder der Schneidvorgang kommt ganz zum Erliegen. Ist die Hochfrequenzleistung dagegen zu hoch eingestellt, entsteht zwischen Chirurgiesonde und dem Gewebe ein kräftiger Lichtbogen. Dieser Lichtbogen verursacht zum einen eine starke Nekrotisierung des Gewebes, wodurch der Heilungsprozeß beeinträchtigt wird. Ein zu stark ausgeprägter Lichtbogen hat aber noch weitere Nachteile. Der wesentliche Nachteil ist eine teilweise Gleichrichtung des Hochfrequenzstromes durch den Lichtbogen, was die Gefahr von Nerven- und Muskelreizungen beim Patienten hervorruft. Solche Muskel- und Nervenreizungen können zu plötzlichen, unvorhersehbaren Bewegungen des Patienten führen, selbst wenn sich dieser in Vollnarkose befindet. In diesem Falle kann der Chirurg nicht mehr kontrolliert arbeiten und es besteht für den Patienten ein hohes Verletzungsrisiko durch die Chirurgiesonde. Durch zu starken Lichtbogen wird zudem das Gewebe zersetzt und bei Unterwasserschnitten, wie z.B. in der Urologie, kann sogar die Spülflüssigkeit thermisch dissoziiert werden. Durch beide Prozesse entstehen explosive Gasgemische, die bei Operationen in Körperhöhlungen zu gefährlichen Explosionen im Körper des Patienten führen können.

Die zum Schneiden notwendige Leistung und das Ausmaß des entstehenden Lichtbogens ist dabei zusätzlich von einer Vielzahl von äußeren Parametern geprägt. Haupteinflußgrößen sind z.B.

- die spezifische elektrische Leitfähigkeit des momentan geschnittenen Gewebes, die einerseits von der Gewebeart und andererseits vom Austrocknungsgrad des Gewebes abhängt,
- die momentane Schnittgeschwindigkeit,
- die momentane Schnittiefe,
- die Form der Chirurgiesonde,
- die Abmessungen der Chirurgiesonde,
- die spezifische elektrische Leitfähigkeit einer eventuell vorhandenen Spülflüssigkeit, die sich durch einschwemmtes Blut und eingeschwemmte Elektrolyte auch während eines Schnittes verändern kann,
- die am Operationsort vorhandene Geometrie bzw. die dortige Verteilung von hochohmigen und niederohmigen Gewebeteilen,
- die momentane Stromdichteverteilung im Körper des Patienten, die sich insbesondere beim Zünden eines Lichtbogens zwischen Chirurgiesonde und zu schneidendem Gewebe extrem schnell und stark ändern kann.

Eine Regelung einer der charakteristischen Kenngrößen des Hochfrequenz-Chirurgiegenerators, das sind z. B.

- der an den Patienten abgegebene Hochfrequenzstrom,
- die an den Patienten angelegte Hochfrequenzspannung,
- die an den Patienten abgegebene Hochfrequenzleistung und
- die am Generator eingestellte Leerlaufspannung,

kann jeweils nur einen Teil, der durch die äußeren Parametern verursachten Einflüsse, ausregeln. So gleicht eine bekannte Regelung der Ausgangsspannung auf einen konstanten Wert den Einfluß von Schnittiefe und Schnittgeschwindigkeit weitgehend aus. Eine geänderte spezifische elektrische Leitfähigkeit des Gewebes, z. B. durch Austrocknung des Gewebes erfordert eine Änderung der Ausgangsspannung, kann also gerade durch eine solche Regelung nicht erfaßt werden.

Die optimale Einstellung des Hochfrequenzgenerators ist dann gegeben, wenn zwischen Chirurgiesonde und Gewebe ein kleiner Lichtbogen besteht, der einerseits für einen zum Schneiden günstigen punktförmigen Übertritt des HF-Stromes von der Chirurgiesonde zum Gewebe sorgt, aber der andererseits die beschriebenen Nachteile eines starken Lichtbogens noch nicht hervorruft.

In dem deutschen Patent DE-C 25 04 280 wird deshalb eine Vorrichtung zum Schneiden und/oder Koagulieren menschlichen Gewebes mit Hochfrequenzstrom beschrieben, bei der eine Anzeigevorrichtung vorhanden ist, die das Ausmaß des zwischen Sonde und Gewebe auftretenden Lichtbogens durch ein elektrisches Signal anzeigt und die eine Regeleinrichtung enthält, die die Stromstärke des an den Patienten abgegebenen Hochfrequenzstromes und damit auch die dem Patienten zugeführte Hochfrequenzleistung so regelt, daß das Ausmaß des Lichtbogens einem voreingestellten Wert entspricht.

Messungen bei Operationen, die mit Chirurgiegeneratoren durchgeführt wurden, deren Leistungseinstellung nach diesem Regelprinzip durchgeführt wird, zeigten deutliche Vorteile gegenüber Operationen mit Generatoren ohne solche Regelungen. Selbst wenn sich die Parameter stark ändern, die sonst noch Einfluß auf die notwendige Generatoreinstellung nehmen, wie elektrische Leitfähigkeit des Gewebes, Austrocknungsgrad des Gewebes, Schnittgeschwindigkeit, Schnittiefe, Form und Abmessungen der Chirurgiesonde usw., kann mit ein und derselben Einstellung des Sollwertes für das Ausmaß des Lichtbogens gearbeitet werden. Die Schnitte sind dabei kaum verschorft, die an den Patienten abgegebene Leistung konnte in einigen Fällen bis auf ein Drittel verringert werden, gegenüber vergleichbaren Operationen mit einem Generator ohne Lichtbogenregelung.

Trotzdem besitzt diese Regelung noch einige Mängel. Sie können beschrieben werden, wenn die physikalischen Effekte näher betrachtet werden, die mit dem beim Schneiden am Operationsort brennenden Lichtbogen verknüpft sind. Der Lichtbogen ist nicht allein von der Leistungsdosierung abhängig. Eine Reihe weiterer physikalischer Effekte wirken auf das Ausmaß des Lichtbogens ein.

Zunächst muß die elektrische Spannung zwischen Chirurgiesonde und Gewebe so hoch sein, daß überhaupt ein Lichtbogen zünden kann. Das erfordert zum einen eine entsprechend hohe Leerlaufspannung des Generators, zum anderen muß aber auch zwischen Chirurgiesonde und Gewebe eine hochohmige oder isolierende Schicht vorhanden sein. Bei verkrusteter Chirurgiesonde wird diese Schicht unter Umständen durch einen Belag aus eingetrockneten Blutkoageln und hängengebliebenen Geweberesten gebildet. Bei kleinem Spalt zwischen Chirurgiesonde und Gewebe bildet Luft oder eine nur gering leitfähige Spülflüssigkeit die hochohmige oder isolierende Schicht. Berührt die Chirurgiesonde das Gewebe und ist ihre Oberfläche sauber, so wird diese hochohmige oder isolierende Schicht durch eine Dampfschicht gebildet, die beim Verdampfen der Zellflüssigkeit entsteht. Die Dicke der entstehenden Dampfschicht hängt dabei von der zugeführten elektrischen Leistung ab.

Die Dicke der hochohmigen oder isolierenden Schicht beeinflußt nun ihrerseits wieder den Lichtbogen und seine Auswirkungen. Je dicker die hochohmige oder isolierende Schicht ist, umso größer wird die Schlagweite des Lichtbogens und umso mehr Leistung wird am Übertrittsort des Lichtbogens in Energie umgesetzt. Hierdurch entsteht ein Teil der beschriebenen Nachteile beim Auftreten eines zu starken Lichtbogens. Mit steigender Schlagweite des Lichtbogens wird zudem der Zusammenhang zwischen HF-Strom im Lichtbogen und HF-Spannung am Lichtbogen immer nichtlinearer. Es steigen dadurch die durch den Lichtbogen in HF-Strom und Spannung entstehenden nichtlinearen Signale, die primär zur momentanen Generatorfrequenz harmonisch sind. Das sind einerseits die Harmonischen 2., 3., 4., und höherer Ordnung, deren Frequenzen das 2-fache, 3-fache, 4-fache, ... der momentanen Frequenz des Ausgangssignals ist, und es ist die Harmonische O.(nullter) Ordnung, die den Gleichrichtereffekt des Lichtbogens beschreibt. Dieser im Lichtbogen entstehende Gleichanteil ist für die Nerven- und Muskelreizungen verantwortlich.

Die Dicke der Dampfschicht folgt als thermischer Effekt nicht sofort der momentan zugeführten Leistung. Das Regelsystem erhält damit eine Totzeit. Das ist besonders beim Anschneiden zu erkennen. Vom Zeitpunkt des Einschaltens des Generators bis zu dem Zeitpunkt zu dem ein Lichtbogen erstmals zündet, vergeht eine nicht vernachlässigbare Zeitspanne, manchmal dauert es einige Sekunden bevor der Schnitt wirklich beginnt. Es ist in der Regelungstechnik bekannt, daß Regelsysteme, die Totzeiten enthalten nur schwer zu stabilisieren sind.

Außerdem brennt der Lichtbogen nicht die ganze Zeit gleichmäßig auf der Oberfläche der Chirurgiesonde. Der Lichtbogen wird, ausreichend hohe Spannung vorausgesetzt, dort zünden, wo die Dampfschicht am dünnsten ist. Die durch den Lichtbogen hervorgerufene starke Energiekonzentration am Übertrittsort des Hochfrequenzstromes verdampft dort die Zellflüssigkeit, die Übertrittsstelle wird dann schnell zu der Stelle mit der dicksten isolierenden Schicht. Der Lichtbogen zündet dann an anderer Stelle. Auf diese Weise tastet der Lichtbogen die gesamte Oberfläche der Chirurgiesonde ab und verdampft letztlich entlang ihrer gesamten Oberfläche die Zellflüssigkeit. An welcher Stelle und mit welcher Schlagweite der Lichtbogen brennt, ist dabei so zufällig, daß das Brennen des Lichtbogens als stochastischer Vorgang betrachtet werden muß. Das hat Auswirkungen auf das Spektrum von Hochfrequenzstrom und Hochfrequenzspannung. So sind z.B. die durch den Lichtbogen entstehenden Spektralanteile in ihrer Amplitude nicht konstant. Die Änderungsgeschwindigkeit reicht dabei bis an eine obere Grenze heran, die durch die Betriebsfrequenz vorgegeben ist. Dadurch entsteht im Frequenzspektrum zusätzlich ein breitbandiges Rauschen, das in EP-A-O 219 568 zur Erkennung des Lichtbogens genutzt wird.

Beeinflussen solche stochastischen Schwankungen die Meßgrößen, die für eine Regelung benutzt werden, so müssen die zufälligen Schwankungen immer durch Mittelwertbildung ausgeglichen werden. Die Messung der stochastischen Vorgänge erfordert somit immer eine endliche Meßzeit. Dies bedeutet wiederum, daß die Regelung nicht beliebig schnell erfolgen kann. Durch die endliche Zeit, die vergeht bis eine eindeutige Regelgröße vorliegt, ist es nicht möglich den Lichtbogen auf einen konstanten Momentanwert zu regeln. Ein zusätzliches Problem bei einer Regelung des Lichtbogens liegt in der bekannten physikalischen Tatsache, daß der im Lichtbogen geltende nichtlineare Zusammenhang zwischen Hochfrequenzspannung und Hochfrequenzstrom stückweise negative Steilheit besitzt, d.h. es ist möglich, daß bei Erhöhung der momentanen Spannung der momentane Strom abnehmen kann und bei Erniedrigung der momentanen Spannung der momentane Strom ansteigen kann. Es ist bekannt, daß solche Vorgänge Schwingungen anregen können und Regelungen entstabilisieren können.

**Darstellung der Erfindung**

Aufgabe der Erfindung ist es nun, die Anordnung zum Schneiden von biologischem Gewebe mit Hochfrequenzströmen nach dem Oberbegriff von Anspruch 1 so auszugestalten, daß trotz der beschriebenen Totzeiten, der notwendigen Mittelwertbildungen und der Gefahr der Entstabilisierung der Regelung durch die physikalischen Effekte des Lichtbogens eine stabile Regelung erreicht wird.

Eine erfindungsgemäße Lösung dieser Aufgabe ist im Patentanspruch 1 angegeben. Weiterbildungen der Erfindung sind in den abhängigen Ansprüchen gekennzeichnet.

Die Erfindung bezieht sich auf eine Anordnung zum Schneiden von biologischem Gewebe mit Hochfrequenzstrom mit einem HF-Generatorteil, das mit einer Chirurgiesonde derart verbunden ist, daß ein Lichtbogen zwischen der Chirurgiesonde und dem zu schneidenen Gewebe erzeugt wird, einer Regeleinrichtung, deren Ausgangssignal an dem HF-Generatorteil anliegt, so daß die Amplitude der an das Gewebe angelegten HF-Spannung oder des HF-Stromes oder der HF-Leistung geregelt wird.

Die Erfindung zeichnet sich durch folgende Merkmale aus:
Die Regeleinrichtung regelt die Ausgangsspannung, den Ausgangsstrom oder die Ausgangsleistung auf einen ersten Sollwert. Es ist eine Anzeigeeinrichtung vorgesehen, deren Ausgangssignal ein Maß für die Größe des erzeugten Lichtbogens ist. Es ist eine Auswerteeinrichtung vorgesehen, an die das Ausgangssignal der Anzeigeeinrichtung und ein zweiter Sollwert für die Größe des Lichtbogens angelegt sind, und die den ersten Sollwert (b) für die Regeleinrichtung wie folgt bildet:
Bei einem Ausgangssignal der Anzeigeeinrichtung, die ein zu großes Ausmaß des Lichtbogens anzeigt, wird der erste Schwellwert um mindestens eine Größenordnung schneller vermindert als er erhöht wird, wenn das Ausgangssignal der Anzeigeeinrichtung den gleichen Betrag der Abweichung nach zu geringen Werten hin besitzt.

Danach wird die Anordnung zur Anzeige des Ausmaßes des Lichtbogens mit einer Regelung mindestens einer der charakteristischen Kenngrößen des Generators entsprechend Patentanspruch 1 kombiniert. Es wird dabei mindestens eine der charakteristischen Kenngrößen des Generators auf einen 1. Sollwert geregelt. Dadurch wird die Auswirkung eines Teiles der äußeren Parameter auf das Schneidverhalten beseitigt. Vorzugsweise wird man die charakteristische Kenngröße auf einen Sollwert regeln, die den äußeren Parameter beeinflußt, der bei der momentan durchgeführten Operationsart am meisten Einfluß auf das Schneidverhalten besitzt. Ändern sich Gewebeart und Austrocknungszustand nur langsam, müssen aber Schnittiefe oder Schnittgeschwindigkeit laufend variiert werden, so ist es günstig die Ausgangsspannung zu regeln.

Bei einer Regelung des Hochfrequenzstromes oder der Hochfrequenzleistung auf einen 1. Sollwert wird der Einfluß der spezifischen elektrischen Leitfähigkeiten auf das Schneidverhalten weitestgehend eliminiert, es bleibt in diesem Falle der Einfluß von Schnittiefe und Schnittgeschwindigkeit auf das Schneidverhalten.

Diese bei der jeweiligen Regelung der charakteristischen Kenngröße des Generators nicht beeinflußbaren Wirkungen äußerer Parameter, werden dadurch ausgeglichen, daß der 1. Sollwert nicht konstant ist, sondern aus einem Vergleich des elektrischen Signales einer Anzeigeeinrichtung für das Ausmaß des Lichtbogens mit einem 2. Sollwert gewonnen wird. Die Gewinnung des 1. Sollwertes erfolgt dazu in einer Auswerteeinrichtung, der einerseits das elektrische Ausgangssignal der Anzeigeeinrichtung für das Ausmaß des Lichtbogens zugeführt ist und andererseits der 2. Sollwert zugeführt ist. Für eine stabile Regelung ist es dabei notwendig, daß sich der in der Auswerteeinrichtung generierte 1. Sollwert für die Regeleinrichtung um mindestens eine Größenordnung langsamer ändert, als die Regeleinrichtung Zeit benötigt, die charakteristische Kenngröße auf den Sollwert einzuregeln.

Kurzfristige Änderungen der äußeren Parameter werden somit durch die schnell arbeitende Regelung der charakteristischen Kenngröße in ihrer Wirkung auf das Schneidverhalten ausgeregelt. Über längere Zeit hinweg gemittelt, ist das Ausmaß des Lichtbogens konstant und durch den 2. Sollwert bestimmt.

Der 2. Sollwert für das Ausmaß des Lichtbogens wird von einem Sollwertgeber geliefert. Im einfachsten Fall gibt der Sollwertgeber einen fest eingestellten Sollwert ab. Meist wird der operierende Arzt noch Einfluß auf den vom Sollwertgeber abgegebenen 2. Sollwert nehmen können und dem Operationsziel anpassen können. Sehr kleine 2.Sollwerte für das Ausmaß des Lichtbogens führen zu Schnitten mit minimaler Nekrose und minimalen Muskel- und Nervenreizungen. Diese Einstellung wird man immer wählen, wenn z. B. in der Nähe von Nervenzentren geschnitten wird und die Gefahr besteht, daß der Patient durch Reizen dieser Nerven zuckt. Solche plötzlichen Bewegungen des Patienten erschweren die Operation und beinhalten die Gefahr, daß der Arzt zu tief schneidet und dabei den Patienten ernsthaft verletzten kann.

Bei Operationen bei denen viel Gewebe entfernt wird, z. B. bei Prostatektomien bis zu 100 g Prostatagewebe, ermöglicht eine höhere Einstellung des Sollwertes für das Ausmaß des Lichtbogens ein zügiges Schneiden. Da zu Beginn dieser Operationen Gewebe in mehreren Schichten abgetragen wird, stört bei den oberen Schichten eine erhöhte Nekrose zunächst nicht, da im weiteren Verlauf der Operation auch diese nekrotisierten Gewebeteile entfernt werden.

Für die Konstruktion der Anordnung zum Schneiden biologischen Gewebes ergeben sich durch die erfindungsgemäß Kombination von Regeleinrichtung für eine der charakteristischen Kenngrößen des Generatorteiles mit der Anzeigeeinrichtung für das Ausmaß des Lichtbogens nach Anspruch 1 weitere Vorteile.

So muß die Regelung der charakteristischen Kenngröße nicht exakt erfolgen, geringfügige Regelabweichungen werden über längere Zeit durch das Nachziehen des 1. Sollwertes ausgeglichen.

Außerdem ist damit nicht notwendig, daß als geregelte charakteristische Kenngröße, unmittelbar eine der Ausgangssignale des Generators wie Ausgangsspannung, Ausgangsstrom, Ausgangsleistung, Leerlaufspannung usw. benutzt wird. Vielmehr können auch charakteristische Kenngrößen, die innerhalb des Generatorteiles auftreten, geregelt werden, wenn sie nur einen eindeutigen Zusammenhang mit den Ausgangsgrößen des Generators besitzen. So genügt es z. B. bei einer Leistungsendstufe des Generatorteiles, die so ausgestaltet ist, daß sie geringen Innenwiderstand besitzt und somit ihre Ausgangspannung bis auf einen nicht allzugroßen Spannungsabfall an diesem Innenwiderstand näherungsweise proportional zur Spannung ihrer Gleichstromversorgung ist, diese Gleichspannung zu regeln. Der Schaltungsaufwand für die Regeleinrichtung kann sich in einem solchen Falle wesentlich vermindern.

Die Kombination der Regeleinrichtung für eine der charakteristischen Kenngrößen mit der Anzeigeeinrichtung für das Ausmaß des Lichtbogens nach Anspruch 1 ermöglicht es auch, für die Anzeigeeinrichtungen physikalische Verfahren zu nutzen, die für eine direkte Regelung für konstantes Ausmaß des Lichtbogens zu langsam sind. So wird in der Patentschrift des deutschen Patentes DE 25 04 280 als besonderer Vorteil der Regelung auf die 3. und höhere Harmonische beschrieben, daß nur diese Harmonische eine schnelle Regelung zulassen. Müssen durch die Regelung auf einen konstanten Sollwert für das Ausmaß des Lichtbogens nur Regelabweichungen und langsam sich ändernde Vorgänge ausgeregelt werden, kann auch die besonders einfach zu messende O. Harmonische zur Anzeige des Ausmaßes des Lichtbogens mit hinreichendem Erfolg genutzt werden. Wird beispielhaft die Anzeige des Ausmaßes des Lichtbogens über die O. Harmonische mit einer Regeleinrichtung für die Ausgangsspannung des Generatorteiles kombiniert, so ergibt sich folgender Wirkungsmechanismus der Gesamtanordnung: Für unterschiedliche Schnittgeschwindigkeit und Schnittiefe erreicht die Konstantregelung der Ausgangsspannung nahezu konstante Schneidbedingungen und konstantes Ausmaß des Lichtbogens. Eine Nachführung der Ausgangs-Spannung ist nur notwendig, wenn sich die Leitfähigkeit des Gewebes am Opertionsort ändert. Dies geschieht entweder, wenn der Schnitt in ein Gewebegebiet mit andersartigem Gewebe führt, z.B. von Muskelgewebe in Fettgewebe, oder wenn durch stetige Erwärmung das Operationsgebiet langsam austrocknet. In diesem Falle wird bei zunächst konstanter Ausgangsspannung das mittlere Ausmaß des Lichtbogens verringert werden. Als Folge davon nehmen die niederfrequenten Anteile und vor allem der Richtstrom ebenfalls ab. Das Ausgangssignal des Lichtbogenregelung wird geringer, die Auswerteeinrichtung erhöht den 1. Sollwert für die Regeleinrichtung bis das Signal der Anzeigeeinrichtung wiederum gleich dem 2. Sollwert für das Ausmaß des Lichtbogens ist.

Wird für die Anzeige des Lichtbogens ein physikalischer Effekt benutzt, der eine schnelle Erkennung des Ausmaß des Lichtbogens zuläßt, wie etwa die Auswertung der im Generatorstrom enthaltenen Harmonischen höherer Ordnung, so muß die Auswerteeinrichtung die Änderungsgeschwindigkeit ihres Ausgangssignales, also dem der Regeleinheit zugeführten 1. Sollwert durch geeignete Maßnahmen so begrenzen, daß die Anstiegsgeschwindigkeit mindestens um eine Größenordnung geringer ist, als die Regelgeschwindigkeit der Regeleinrichtung.

In einer besonders günstigen Ausgestaltung der Erfindung erfolgt die Begrenzung der Änderungsgeschwindigkeit dadurch, daß zunächst die momentane Abweichung des Ausgangssignales der Anzeigeschaltung für das Ausmaß des Lichtbogens mit dem 2. Sollwert verglichen wird. Eine mögliche schaltungstechnische Realisation dieses Vergleiches kann durch Bildung der Differenz beider Signale, vorzugsweise mit einem Differenzverstärker erfolgen. Das damit gebildete Differenzsignal wird sich zunächst noch schnell ändern. Wird nun der zeitliche Mittelwert dieses Signales gebildet, ist dieses Ausgangssignal geeignet, als 1. Sollwert der Regeleinrichtung zugeführt zu werden. Schaltungen zur Mittelwertbildung sind allgemein bekannt. Die einfachste Realisierung besteht in einem RC-Tiefpaß mit definierter Grenzfrequenz $f_{g1}$.

In einer weiteren vorteilhaften Ausführung der Auswerteeinrichtung wird ebenfalls ein Differenzsignal aus dem Ausgangssignal der Anzeigevorrichtung für das Ausmaß des Lichtbogens und dem 2. Sollwert für das Ausmaß des Lichtbogens gebildet. Dieses Ausgangssignal wird einer Schaltung mit zeitlich integrierender Wirkung zugeführt, wie sie z. B. in bekannter Weise mit Hilfe eines kapazitiv rückgekoppelten Differenzverstärkers realisiert werden kann. In diesem Falle wird sich das Ausmaß des Lichtbogens so lange verändern, bis ohne bleibende Regelabweichung das Ausgangssignal der Anzeigevorrichtung für das Ausmaß des Lichtbogens gleich dem 2. Sollwert für das Ausmaß des Lichtbogens ist. Ein eventuell notwendiger Gleichspannungsoffset für den 1. Sollwert, der der Regeleinrichtung zugeführt wird, stellt sich durch diese Ausgestaltung automatisch ein.

Durch Mittelwertbildung oder Integration wird der 1. Sollwert nur langsam verändert. Wegen der bereits beschriebenen Totzeiten bis zum Zünden eines Lichtbogens ist es meist zweckmäßig das Ausgangssignal des Generators nur sehr langsam zu erhöhen. Wenn der Lichtbogen dann auftritt, ist trotz der Totzeiten der 1. Sollwert nur geringfügig größer, als es im eingeschwungenen Zustand der Regelung optimal wäre. Das Ausmaß des Lichtbogens ist dann ebenfalls nahe dem Optimum. Die Regelgeschwindigkeit des 1. Sollwertes für eine höhere charakteristische Kenngröße des Generatorteiles wird in diesem Falle um mindestens eine Größenordnung geringer sein als die Regelgeschwindigkeit der Regelvorrichtung. Tritt in diesem Falle ein zu hoher Lichtbogen auf, entweder dadurch daß sich die äußeren Parameter geändert haben oder weil die Totzeit so lange gedauert hat, daß der der Regeleinrichtung zugeführte 1. Sollwert doch weit über den optimalen Wert angestiegen ist, so würde der dann zu stark brennende Lichtbogen die bereits beschriebenen Nachteile hervorrufen. In diesem Falle wird die Auswerteeinrichtung so ausgebildet, daß die Änderungsgeschwindigkeit des 1. Sollwertes in die Richtung, die ein Herunterregeln der geregelten charakteristischen Kenngröße des Generatorteils bedeutet, um mindestens eine Größenordnung größer ist als beim Hochregeln.

Ein besonderes Problem bei einer Regelung, die das Ausmaß des Lichtbogens konstant hält, ist die Zeit die zwischen Generatoraktivierung und dem 1. Zünden des Lichtbogens vergeht. Zu diesem Zeitpunkt befindet sich das zu schneidende Gewebe auf Körpertemperatur. Nun muß zunächst das Gewebe auf Siedetemperatur der Zellflüssigkeit hochgeheizt werden und dann muß genügend Zellflüssigkeit verdampft werden, bis Chirurgiesonde und Gewebe vollständig durch eine Dampfschicht voneinander isoliert sind, erst dann kann der Lichtbogen zünden. Messungen zeigen, daß in diesem Falle zwischen Generatoraktivierung und dem Zünden des Lichtbogens mehrere Sekunden vergehen können, vor allem dann, wenn die Chirurgiesonde bei der Generatoraktivierung fest auf das Gewebe aufgepreßt ist. In diesem Falle steigt während der gesamten Zeit, in der kein Lichtbogen gezündet hat, der 1. Sollwert für die Regeleinrichtung zur Regelung einer der charakteristischen Kenngröße des Generators stetig an. Ohne besondere Maßnahme brennt dann, wenn erst der Lichtbogen gezündet hat ein viel zu großer Lichtbogen. Dieser Nachteil kann vermieden werden, wenn der 1. Sollwert durch eine geeignete Schaltung nicht über einen vorgegebnen Grenzwert ansteigen kann. So zeigen Messungen, daß z.B. in der Zahnheilkunde keine Spannungen mit einem Effektivwert höher als 250 V benötigt werden, wenn der zur Operation benötigte Strom wieder niederohmig zum Generator zurückgeführt wird. In diesem Falle ist es günstig, die Ausgangsspannung des Generators als charakteristische Kenngröße auf einen konstanten Wert zu regeln aber keine höheren Sollwerte für die Regeleinrichtung zuzulassen, als es einer Ausgangsspannung von 250 V entspricht.

Für die charakteristische Kenngröße des Generators, die durch die Regeleinrichtung auf den 1. Sollwert geregelt wird, kann häufig auch eine untere, für die Anwendung sinnvolle Schranke angegeben werden. So sind für die Amplitude der an das Gewebe angelegten Spannung Werte von mindesten 150 V notwendig, damit überhaupt ein Lichtbogen zünden kann. In diesem Falle empfiehlt es sich, den Sollwert für die Regeleinrichtung so zu begrenzen, daß der für die Anwendung sinnvolle Grenzwert der charakteristischen Kenngröße nicht unterschritten wird und natürlich der sinnvolle obere Wert nicht überschritten wird.

In diesem Falle ist der notwendige Regelbereich für die Regelung der charakteristischen Kenngröße des Generators eingeschränkt, es lassen sich damit höhere Regelgeschwindigkeiten und eine höhere Stabilität der Regelung erreichen.

In einer weiteren vorteilhaften Ausgestaltung der Erfindung, wird das Problem der manchmal langen Zeitverzögerung von Generatoraktivierung bis zum Zünden des Lichtbogens dadurch vermieden, daß die Auswerteeinrichtung um einen Schaltungsteil erweitert ist, der erkennt ob ein Lichtbogen gezündet hat oder nicht. In einfachster Form besteht dieser Schaltungsteil aus einem Komparator, der anzeigt, ob des Ausgangssignal der Anzeigeeinrichtung für das Ausmaß des Lichtbogens von Null verschieden ist. Ist das Ausgangssignal der Anzeigeeinrichtung gleich Null, so ist kein Lichtbogen vorhanden. Als 1. Sollwert für die Regeleinrichtung wird in diesem Falle ein fest eingestellter Sollwert weitergegeben. Erst wenn der Lichtbogen gezündet hat, wird der 1. Sollwert aus einem Vergleich des zweiten Sollwertes mit dem Ausgangssignal der Anzeigeeinrichtung für das Ausmaß des Lichtbogens gebildet. Natürlich wird man bei der praktischen Realisierung die Schaltung so aufbauen, daß Mittelwertbildung oder Integrationsschaltung in der Zeit ohne Lichtbogen nicht in eine Begrenzung laufen. Das Einschwingen der Regelung würde sonst unnötig verlangsamt.

Bei manchen Operationen aktiviert der Arzt den Generator bereits eine Zeit bevor er mit der Chirurgiesonde das Gewebe berührt. Das wird insbesondere der Fall sein, wenn er nur wenig Gewebe abzutragen hat, dabei aber besonders vorsichtig vorgehen muß. In diesem Falle ist die Zeit zwischen Generatoraktivierung und dem Auftreten eines Lichtbogens nochmals verlängert. Der Zustand, daß die Chirurgiesonde das Gewebe noch nicht berührt hat, läßt sich durch eine Überwachung der zwischen Chirurgiesonde und Gewebe auftretenden Impedanz Z, erkennen. Deshalb wird in einer weiteren vorteilhaften Ausführung die Anordnung zum Schneiden von biologischem Gewebe durch eine Schaltung zur Ermittlung der momentanen Impedanz ergänzt und deren Ausgangssignal ebenfalls der Auswerteeinrichtung zugeführt. In Verbindung mit dem Ausgangssignal der Anzeigevorrichtung für das Ausmaß des Lichtbogens kann dann der 1. Sollwert noch besser an das Operationsgeschehen angepaßt werden.

In einer besonders vorteilhaften Ausgestaltung der Erfindung wird dabei der 1. Sollwert für die Regeleinrichtung solange auf einen voreingestellten niedrigen Wert gesetzt, solange durch eine hochohmige Impedanz Z angezeigt ist, daß die Chirurgiesonde das Gewebe noch gar nicht berührt. Erst wenn die Impedanz Z einen voreingestellten Grenzwert Zu unterschreitet, wird die charakteristische Kenngröße des Generators auf die sonst von der Auswerteeinrichtung vorgegebene größere eingestellt. Der Grenzwert Zu hängt dabei von der Anwendung der Anordnung ab. Ist die beschriebene Anordnung Bestandteil eines HF-Chirurgiegenerators für die Zahnmedizin, so zeigen Messungen der Erfinder, daß bei einer Generatorfrequenz von 350 kHz ohne Gewebeberührung die Belastungsimpedanz des HF-Generators bei einer Operationsanordnung mit angelegter Ableitelektrode über 20 kΩ beträgt und bei Gewebeberührung deutlich darunter liegt. In diesem Falle ist es sinnvoll als Schwellwert einen Impedanzwert von Zu = 20 kΩ zu wählen.

Die Anzeigevorrichtung für die Belastungsimpedanz muß nicht notwendigerweise einen Analogwert an die Auswerteeinrichtung weiterreichen. Die Entscheidung ob die Chirurgiesonde das Gewebe berührt oder nicht kann bereits in der Anzeigevorrichtung zur Bestimmung der Belastungsimpedanz getroffen werden. In diesem Falle kann die sonst zur Impedanzbestimmung notwendige Quotientenbildung von Generatorspannung U durch Generatorstrom I ( Z = U/I ) entfallen. Es genügt dann einen zum Generatorstrom I proportionalen Wert $v_1$*I und einen zur Generatorspannung proportionalen Wert $v_2$*U an einen Komparator zu geben. Das Ausgangssignal des Komparators wird genau dann seinen Schaltzustand ändern, wenn $v_1$*I gleich $v_2$*U ($v_1$*I = $v_2$*U). Damit ist der Grenzwert für die Umschaltung Zu gegeben durch Zu = $v_2/v_1$. Die Faktoren $v_1$ und $v_2$ können mit bekannten Verfahren z.B. durch Spannungsteilerschaltungen leicht eingestellt werden. Das Ausgangssignal des Komparators besitzt dann nur die zwei Schaltzustände, die anzeigen, ob die momentane Belastungsimpedanz Z der Anordnung zum Schneiden von biologischem Gewebe größer oder kleiner als Zu ist. Dieses Signal kann dann als Ausgangssignal der Anzeigevorrichtung für die Belastungsimpedanz der Auswerteeinrichtung zugeführt werden. Dort kann es ohne oder mit nur geringer Weiterverarbeitung als Umschaltsignal von einem vorgegebenen niedrigen Wert, auf den aus der Anzeigevorrichtung für das Ausmaß des Lichtbogens ermittelten variablen Wert als 1. Sollwert für die Regeleinrichtung dienen.

Für die praktische Ausführung der Auswerteeinrichtung können alle bekannten analog arbeitenden Schaltungen, die die in den Patentansprüchen definierten Eigenschaften besitzen, verwendet werden. Da der 1. Sollwert, der der Regeleinrichtung für einen charakteristischen Kennwert des HF-Generatorteiles von der Auswerteeinrichtung zugeführt ist, sich nur langsam zu ändern braucht, kann die Auswerteeinrichtung in einer besonders vorteilhaften Ausführung auch durch digitale Schaltglieder realisiert sein. Neu auf dem Markt befindliche HF-Chirurgiegeneratoren enthalten zum Teil bereits Mikroprozessoren. In diesem Falle kann die Gesamtanordnung besonders einfach an die unterschiedlichen Operationsaufgaben angepaßt werden. Es genügt dann zum Erreichen unterschiedlicher Charakteristiken oder unterschiedlicher Grenzwerte das Programm des Mikroprozessors zu ändern.

**Kurze Beschreibung der Zeichnungen**

Die Erfindung wird nachstehend ohne Beschränkung des allgemeinen Erfindungsgedanken anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

Fig. 1: Prinzipschaltbild der Anordnung zum Schneiden von biologischem Gewebe entsprechend Anspruch 1,

Fig. 2: Prinzipschaltbild einer Ausgestaltung der Auswerteeinrichtung mit Differenzbildung und Mittelwert bildung,

Fig. 3: Prinzipschaltbild einer Ausgestaltung der Auswerteeinrichtung mit Differenzbildung und Integration,

Fig. 4: Prinzipschaltbild einer Ausgestaltung der Auswerteeinrichtung mit unterschiedlichen Zeitkonstante für die Regelung in Aufwärtsrichtung bzw. in Abwärtsrichtung mit Diagrammen der Zeitverläufe der Regelsignale,

Fig. 5: Prinzipschaltbild einer Ausgestaltung der Auswerteeinrichtung mit Begrenzung des Sollwertes für die Regeleinrichtung auf einen maximalen Grenzwert,

Fig. 6: Prinzipschaltbild einer Ausgestaltung der Auswerteeinrichtung mit Begrenzung des Sollwertes für die Regeleinrichtung auf einen maximalen oberen Grenzwert und auf einen minimalen unteren Grenzwert,

Fig. 7: Prinzipschaltbild einer Ausgestaltung der Auswerteeinrichtung mit Umschalten des Sollwertes für die Regeleinrichtung auf einen voreingestellten festen Wert B1 solange kein Lichtbogen erkannt ist,

Fig. 8: Prinzipschaltbild der Anordnung zum Schneiden von biologischen Gewebe entsprechend Anspruch,

Fig. 9: Prinzipschaltbild einer Ausgestaltung der Auswerteeinrichtung mit Umschalten des Sollwertes für die Regeleinrichtung auf einen voreingestellten festen Wert B2 solange die Belastungsimpedanz Z kleiner ist als ein vor eingestellter Grenzwert Z.

## Beschreibung eines Ausführungsbeispiels

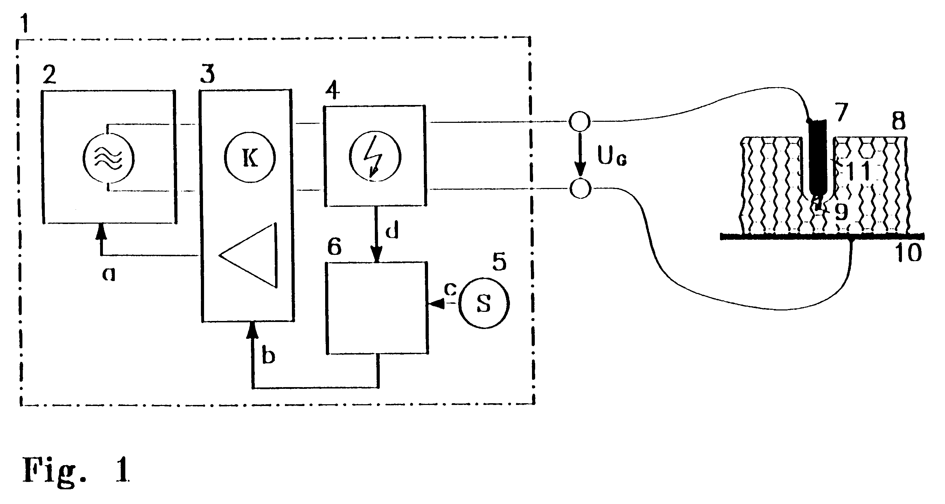

In Fig. 1 ist das Prinzipschaltbild der Anordnung zum Schneiden von biologischem Gewebe (1) gemäß Anspruch 1 zusammen mit der am häufigsten verwendeten Ankopplung des Gewebes an die Anordnung dargestellt. Die eine Klemme der Anordnung ist leitend mit der Chirurgiesonde (7) verbunden. Die Chirurgiesonde wird häufig auch Aktive Elektrode oder Schneidelektrode genannt. Die zweite Klemme ist meist leitend mit einer zweiten, großflächigen Elektrode (10), die in der Regel abseits des Operationsortes angebracht ist, verbunden. Diese zweite Elektrode wird häufig als Ableitelektrode, Neutrale Elektrode oder Passive Elektrode bezeichnet. Zwischen der Chirurgiesonde (7) und der zweiten Elektrode befindet sich das zu schneidende biologische Gewebe (8). Während des Schnittes bildet sich zwischen Chirurgiesonde (7) und Gewebe (8) eine hochohmige oder isolierende Schicht (11), die durch einen Lichtbogen (9) durchschlagen wird. Die Anordnung zum Schneiden von biologischem Gewebe gemäß Anspruch 1 besteht aus einem Generatorteil (2), durch den die zum Schneiden notwendige HF-Leistung erzeugt wird. Für die Anordnung zum Schneiden von biologischem Gewebe benötigt man dabei ein Generatorteil (2) dessen Ausgangsleistung durch ein elektronisches Signal (a) verändert werden kann. Dabei ist es für die Erfindung nicht von Bedeutung, welche der Ausgangsgrößen des Generatorteiles wie Ausgangsspannung, Ausgangsstrom, Ausgangsleistung, Leerlaufspannung primär durch das Signal a beeinflußt wird. Über die Generatorscharakteristik und die durch die äußere Beschaltung festgelegte Impedanz sind diese Größen alle eindeutig miteinander verknüpft.

In der Anordnung zum Schneiden von biologischem Gewebe ist als weitere Baugruppe eine Einrichtung zum Regeln mindestens einer der charakteristischen Kenngrößen des Generators vorhanden. Das Signal (b) stellt in dieser Anordnung einen 1. Sollwert dar, auf den die charakteristische Größe (K) geregelt wird. In der Zeichnung ist die Einrichtung zum Regeln der charakteristische Größe so eingezeichnet, daß eine Größe (K), die am Ausgang des Generatorteiles erfaßbar ist, geregelt wird. Statt dessen kann als charakteristische Größe (K) auch nur eine im Generatorteil auftretende Größe geregelt werden, wenn diese einen eindeutigen Zusammenhang mit den Ausgangsgrößen des Generatorteiles besitzt. Der 1. Sollwert (b) der in der Anordnung zum Schneiden von biologischem Gewebe auftritt, ist kein fest eingestellter Wert, sondern er wird durch die nachfolgend beschriebenen Teile der Gesamtanordnung eingestellt. Dazu ist zunächst eine Anzeigevorrichtung (4) vorhanden, die das Ausmaß des zwischen Chirurgiesonde (7) und Gewebe (8) brennenden Lichtbogens (9) mit einem elektrischen Signal (d) anzeigt. Als Anzeigevorrichtungen können alle bis jetzt bekannt gewordenen Schaltungen zur Erkennung eines Lichtbogens mit elektrischen Signalen dienen, also insbesondere auch alle in der Deutschen Patentschrift 2504280 beschriebenen Methoden. Das Ausgangssignal (d) der Anzeigevorrichtung (4) für das Ausmaß des Lichtbogens ist einer Auswerteeinrichtung (6) zugeführt, die daraus und einem von einem Sollwertgeber (5) vorgegebenen Sollwert c den 1. Sollwert (b) für die Einrichtung zum Regeln mindestens einer der charakteristischen Größen des Generatorteiles bildet. Für die Funktion der Gesamtanordnung ist es wichtig, daß sich der 1. Sollwert (b) mindestens eine Größenordnung langsamer verändert als die Regeleinrichtung zum Einregeln der charakteristischen Kenngröße benötigt. Mögliche Ausgestaltungen für die Auswerteeinrichtung (6) sind in den Unteransprüchen und der dazugehörigen Beschreibung beschrieben und werden in mit den nächsten Figuren näher erläutert.

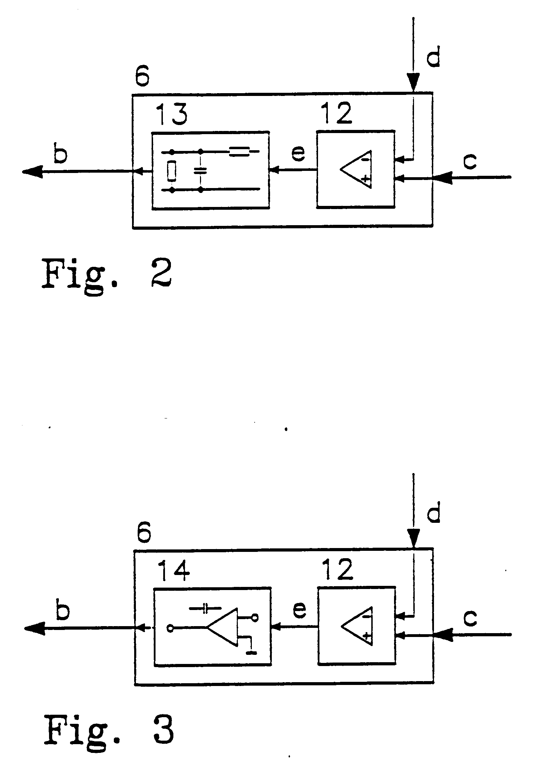

In Fig. 2 ist eine vorteilhafte Ausgestaltung der Auswerteeinrichtung (6) schematisch dargestellt. Aus den Eingangssignalen (d), (c) der Auswerteeinrichtung (6) wird durch eine differenzbildende Schaltung (12) das Differenzsignal e gebildet. Für die Realisierung der Schaltung sind dem Fachmann viele Möglichkeiten bekannt, eine davon ist die dargestellte Schaltung mit einem Operationsverstärker. Anschließend wird durch die Schaltung (13) der Mittelwert des Differenzsignales (e) gebildet. Die einfachste Form einer linearen Mittelwertbildung kann wie angegeben durch einen RC-Tiefpaß erfolgen.

Andere Schaltungen mit Tiefpaßfunktionen sind zur Lösung dieser Aufgabenstellung ebenso geeignet, insbesondere auch aktive Tiefpässe. Bei ihnen ist die normalerweise noch nötige Addition eines Offsetwertes zum Ausgangssignal besonders einfach zu lösen. Die Mittelwertbildung ist allerdings nicht auf den linearen Mittelwert, wie ihn Tiefpässe bilden beschränkt. Besonders vorteilhaft kann es sein, den quadratischen MITTELWERT des Differenzsignales zu bilden. In diesem Falle steigt dann allerdings der Schaltungsaufwand erheblich.

In Fig. 3 ist eine weitere vorteilhafte Ausgestaltung der Auswerteeinrichtung (6) schematisch dargestellt. Aus den Eingangssignalen (d), (c) der Auswerteeinrichtung (6) wird wie in Fig. 2 durch eine differenzbildende Schaltung (12) das Differenzsignal e gebildet. Danach folgt eine Schaltung mit integrierender Wirkung (14), wie sie z. B. mit Hilfe eines kapazitiv rückgekoppelten Operationsverstärkers realisiert werden kann. Die besonderen Vorteile dieser Schaltungsanordnung bestehen darin, daß das Ausmaß des Lichtbogens ohne bleibende Regelabweichung auf den 2. Sollwert (c) geregel wird, selbst wenn der 1. Sollwert (b) einen mit der Zeit oder der Betriebstemperatur der Schaltung veränderlichen Offsetwert aufweisen muß.

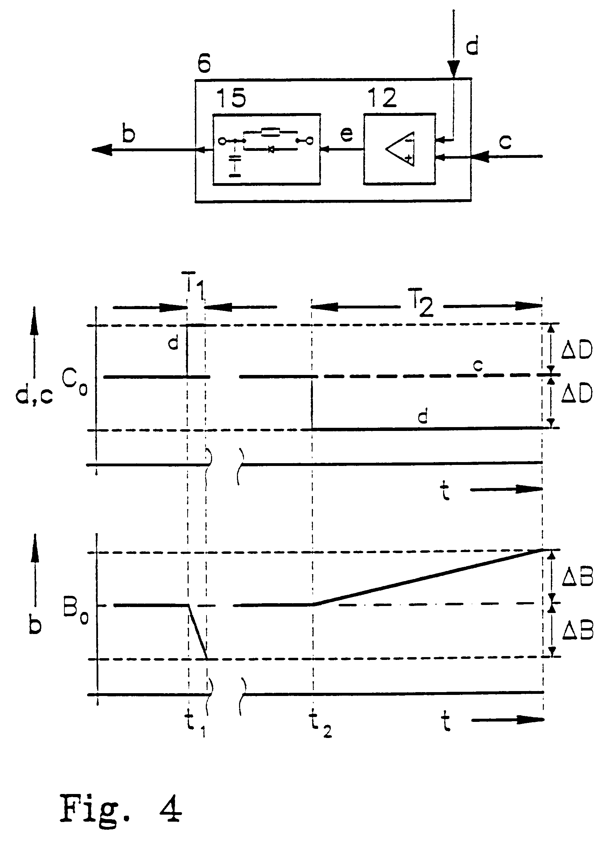

In Fig. 4 ist eine weitere vorteilhafte Ausgestaltung der Auswerteeinrichtung (6) schematisch dargestellt. Aus den Eingangssignalen (d), (c) der Auswerteeinrichtung (6) wird wie in Fig. 2 und Fig 3. durch eine differenzbildende Schaltung (12) das Differenzsignal e gebildet. Danach folgt eine Schaltung mit Tiefpaßverhalten (15) und/oder mit integrierender Wirkung. Zum Unterschied zu den in Fig. 2 oder Fig. 3 angegebenen Schaltungen besitzt diese Schaltung unterschiedliche Zeitkonstanten, je nachdem ob das Signal (d) das das Ausmaß des Lichtbogens anzeigt größer oder kleiner ist als der 2. Sollwert (c). Die einfachste Möglichkeit, solche unterschiedlichen Zeitkonstanten zu realisieren besteht darin, das speichernde Element der Schaltung zur Mittelwertbildung oder der Integrationsschaltung über unterschiedliche Widerstände an die vorhergehende Schaltung anzukoppeln. Die Umschaltung kann wie in Fig. 4 schematisch angedeutet durch eine Diode (D1) geschehen, die gleiche Funktion könnte aber auch über gesteuerte Schalter erreicht werden.

Die Zeitdiagramme in Fig. 4 sollen die Funktion der Schaltung näher erläutern. Es sind zunächst in einem Diagramm die Eingangssignale (c) und (d) der Auswerteinrichtung (6) eingetragen. Der 2. Sollwert (c), der das gewünschte Ausmaß des Lichtbogens angibt, soll die ganze Zeit über konstant sein ($C_0$). (gestrichelte Linie). Für das Signal 2, das das tatsächliche Ausmaß des Lichtbogens zum Zeitpunkt $t_1$ beschreibt, ist das Ausmaß des Lichtbogens plötzlich größer geworden, und zwar um den Wert D. Als Antwort darauf erniedrigt sich das Ausgangssignal b der Auswerteeinrichtung, das für die nachfolgende Regeleinrichtung (3) den 1. Sollwert darstellt. Aufgrund des Tiefpaßverhaltens der mittelwertbildenten oder integrierenden Schaltung (15) dauert es eine endliche Zeitspanne $T_1$ bis das Ausgangssignal b eine bestimmte Abweichung B vom zuvor angenommenen Wert $B_0$ erreicht hat. Als nächstes ist in den Diagrammen zum Zeitpunkt $t_2$ eine Abweichung D des Signales d vom Wert c = $C_0$ in die Richtung gezeichnet, die ein vermindertes Ausmaß des Lichtbogens angibt. Nun dauert es eine deutlich größere Zeitspanne $T_2$, bis das Signal b die gleiche Abweichung B vom Wert $B_0$ erreicht hat. Erfindungsgemäß soll die Schaltung (15) so ausgestaltet sein, daß die Zeitspanne T2 wesentlich größer ist, als die Zeitspanne $T_1$, also gilt $T_2 \gg T_1$. Die gezeichneten Zeitverläufe der Signale c, d und b sollen nur das prinzipielle Verhalten der Schaltung verdeutlichen, sie sind bei geschlossener Regelschleife in der tatsächlich realisierten Schaltung nicht in der gezeichneten Form meßbar. Jede Änderung des Signales b führt sofort zu einer Änderung der geregelten charakteristischen Kenngröße des Generatorteiles und damit auch zu einer Änderung des Ausmaß des Lichtbogens und zu einer Änderung des Signales d. Eine zeitlich konstante Abweichung des Signales d um den Wert D ist somit bei geschlossener Regelschleife möglich. Die Signale können in ähnlicher Form allerdings gemessen werden, wenn die Regelschleife, wie für prinzipielle Untersuchungen üblich an beliebiger Stelle aufgetrennt wird.

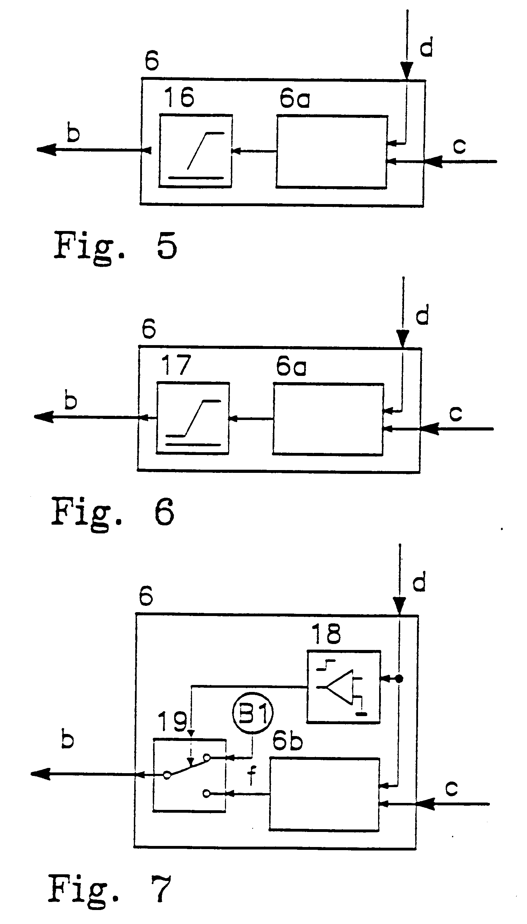

Die Fig. 5 stellt eine weitere vorteilhafte Ausgestaltung der Auswerteeinrichtung dar. Der Schaltungsblock 6a ist dabei eine komplette in den Fig. 1-4 bereits beschriebene Ausgestaltung der Auswerteeinrichtung 6. Bevor das Ausgangssignal als 1. Sollwert (b) an die Regeleinrichtung weitergegeben wird ist nun noch eine Begrenzerschaltung 16 vorhanden, die ein Ansteigen des Signales b über einen Grenzwert $B_{max}$ verhindert.

Die Fig. 6 zeigt eine weitere vorteilhafte Ausgestaltung der Auswerteeinrichtung. Der Schaltungsblock 6a ist dabei eine komplette in den Fig. 1-4 bereits beschriebene Ausgestaltung der Auswerteeinrichtung 6. Bevor das Ausgangssignal als 1. Sollwert (b) an die Regeleinrichtung weitergegeben wird ist nun noch eine Begrenzerschaltung 16 vorhanden, die ein Ansteigen des Signales b über einen oberen Grenzwert $B_{max}$ und ein Absinken unter einen unteren Grenzwert $B_{min}$ verhindert.

Die Fig. 7 stellt eine weitere vorteilhafte Ausgestaltung der Auswerteeinrichtung dar. Der Schaltungsblock 6b ist dabei eine komplette in den Fig. 1-6 bereits beschriebene Ausgestaltung der Auswerteeinrichtung 6. Das Ausgangssignal des Schaltungsteiles (6b) wird nur als 1. Sollwert (b) an die Regeleinrichtung weitergegeben, wenn ein Lichtbogen vorhanden ist. In Zeiten, in denen kein Licht-bogen vorhanden ist, wird für das Signal b der voreingestellte Wert B1 durch den Umschalter (19) angelegt. Die Entscheidung über das Umschalten trifft der Schaltungsteil (18), der in einfachster Ausführung aus einem Komparator besteht, der feststellt, ob das Ausmaß des Lichtbogens, das durch das Signal d angezeigt wird, von Null verschieden ist.

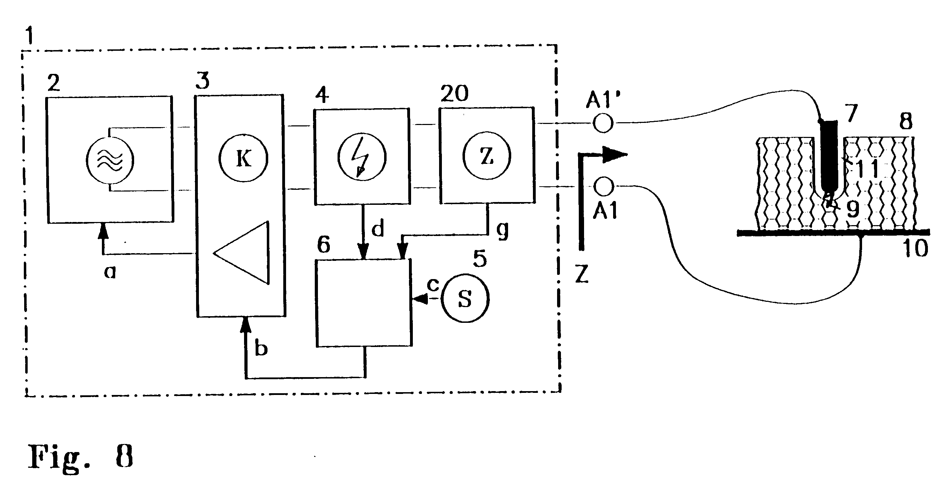

Mit Fig. 8 wird eine weitere vorteilhafte Ausführung der Erfindung beschrieben. Fig. 8 stellt dabei nochmals schematisch die gesamte Anordnung zum Schneiden von biologischem Gewebe dar. Zusätzlich zu den in Fig. 1 dargestellten Funktionseinheiten erscheint hier die Anzeigeeinrichtung (20) für die Impedanz Z mit ihrem Ausgangssignal g, das ebenfalls der Auswerteeinrichtung (6) zugeführt ist. Die Impedanz Z ist die Belastungimpedanz, die an den Ausgangsklemmen (A1, A1') der Gesamtanordnung durch die Ankopplung an das biologische Gewebe auftritt. Durch Einbeziehen der Impedanz Z in die Bildung des 1. Sollwertes (b) für die Regeleinrichtung, kann noch mehr auf die momentanen Betriebszustände am Operationsort eingegangen werden.

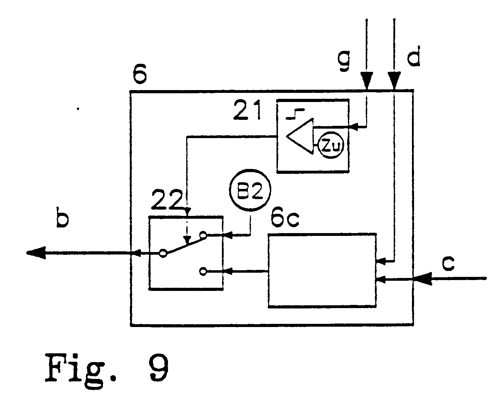

Fig. 9 stellt eine einfache, aber besonders vorteilhafte Ausführung des Auswerteinrichtung (6) dar, die den Wert der momentanen Impedanz Z zusätzlich mit auswertet. Dabei ist der Block 6c eine der in den Fig. 1 bis 7 beschriebenen Ausgestaltungen der Auswerteeinrichtung 6. Zusätzlich ist nun noch eine Vergleicherschaltung vorhanden, die feststellt, ob die momentane Impedanz größer oder kleiner als ein voreingestellter Wert Zu. ist. Ist Z > Zu, so berührt die Chirurgiesonde das Gewebe nicht. In diesem Falle wird, durch den Umschalter (22) ein vermindertet Wert B2 als 1. Sollwert b an die Regeleinrichtung der charakteristischen Kenngröße des Generatorteiles gegeben.

**Patentansprüche**

1. Anordnung zum Schneiden von biologischem Gewebe mit Hochfrequenzstrom (1) mit

 - einem HF-Generatorteil (2), das mit einer Chirurgiesonde (7) derart verbunden ist, daß ein Lichtbogen (9) zwischen der Chirurgiesonde und dem zu schneidenen Gewebe (8) erzeugt wird,
 - einer Regeleinrichtung (3), deren Ausgangssignal an dem HF-Generatorteil anliegt, so daß die Amplitude der an das Gewebe angelegten HF-Spannung oder des HF-Stromes oder der HF-Leistung geregelt wird,
 **gekennzeichnet** durch folgende Merkmale:
 - die Regeleinrichtung (3) regelt die Ausgangsspannung, den Ausgangsstrom oder die Ausgangsleistung auf einen ersten Sollwert (b),
 - es ist eine Anzeigeeinrichtung (4) vorgesehen, deren Ausgangssignal ein Maß für die Größe des erzeugten Lichtbogens ist,
 - es ist eine Auswerteeinrichtung vorgesehen, an die das Ausgangssignal der Anzeigeeinrichtung und ein zweiter Sollwert für die Größe des Lichtbogens angelegt sind, und die den ersten Sollwert (b) für die Regeleinrichtung wie folgt bildet:
 bei einem Ausgangssignal der Anzeigeeinrichtung, die ein zu großes Ausmaß des Lichtbogens anzeigt, wird der erste Sollwert um mindestens eine Größenordnung schneller vermindert als er erhöht wird, wenn das Ausgangssignal der Anzeigeeinrichtung den gleichen Betrag der Abweichung nach zu geringen Werten hin besitzt.

2. Anordnung nach Anspruch 1,
 **daddurch gekennzeichnet**, daß die Auswerteeinheit (6) zur Bildung des 1. Sollwertes für die Regeleinrichtung (b) eine Subtraktions-Schaltung (12) enthält, die ein Differenzsignal (e) aus dem Ausgangssignal der Anzeigevorrichtung (d) und dem 2. Sollwert für das Ausmaß des Lichtbogens (c) bildet, sowie eine mittelwertbildende Schaltung (13), die dieses Differenzsignal (e) zeitlich mittelt.

3. Anordnung nach Anspruch 1,
 **dadurch gekennzeichnet**, daß die Auswerteeinheit zur Bildung des 1. Sollwertes für die Regeleinrichtung eine Subtraktions-Schaltung (12) enthält, die ein Differenzsignal (e) aus dem Ausgangssignal der Anzeigevorrichtung (d) und dem 2. Sollwert für das Ausmaß des Lichtbogens (c) bildet, sowie eine Integrationsschaltung (14), die dieses Differenzsignal (e) zeitlich integriert.

4. Anordnung nach einem der Ansprüche 1 bis 3,
 **dadurch gekennzeichnet**, daß die Auswerteeinrichtung eine Begrenzerschaltung (16) enthält, die den Sollwert nicht über einen voreingestellten Grenzwert ansteigen läßt.

5. Anordnung nach Anspruch 4,
 **dadurch gekennzeichnet**, daß die Auswerteeinrichtung eine Begrenzerschaltung enthält, so daß der 1. Sollwert (b) nicht unter einem voreingestellten Grenzwert und nicht über einen voreingestellten 2. Grenzwert liegt.

6. Anordnung nach einem der Ansprüche 1 bis 4,
 **dadurch gekennzeichnet**, daß die Auswerteinrichtung eine Schaltung zur Lichtbogenerkennung (18) besitzt und daß solange ein voreingestellter Wert (B1) eines Gleichsignales (f) als 1. Sollwert an den Ausgang geschaltet ist, solange das Ausgangssignal der Anzeigeeinrichtung (d) anzeigt, daß kein Lichtbogen vorhanden ist und erst beim Auftreten eines Lichtbogens der 1. Sollwert (b) aus dem Vergleich des Ausgangssignales der Anzeigeeinrichtung (d) mit dem 2. Sollwert (c) gebildet wird.

7. Anordnung nach einem der Ansprüche 1 bis 6,
 **dadurch gekennzeichnet**, daß zusätzlich eine Anzeigevorrichtung (20) vorhanden ist, die die momentane Impedanz (Z) anzeigt, mit der die Anordnung zum Schneiden von biologischem Gewebe durch das zu schneidende Gewebe am Ausgang (A1, A1') belastet ist, und daß deren Ausgangsignal (g) ebenfalls der Auswerteeinrichtung (6) zugeführt ist.

8. Anordnung nach Anspruch 7,
 **dadurch gekennzeichnet**, daß die Auswerteeinrichtung (6) einen voreingestellten, niedrigen Wert (B2) als Sollwert (b) einstellt, solange sie aus dem Ausgangssignal (g) der Anzeigevorrichtung zur Bestimmung der momentanen Impedanz erkennt, daß die Impedanz (Z) größer ist, als ein vorgewählter Schwellwert (Zu).

9. Anordnung nach Anspruch 7,
 **dadurch gekennzeichnet**, daß die Anzeigevorrichtung zur Bestimmung der momentanen Impedanz (20) so gestaltet ist, daß ihr Ausgangssignal (g) nur zwei Schaltzustände aufweist, wovon der eine Schaltzustand auftritt, wenn die Belastungsimpedanz (Z) am Ausgang (A1,A1') der Vorrichtung zum Schneiden von biologischem Gewebe größer ist als ein voreingestellter Wert (Zu) und der zweite Schaltzustand eingenommen wird, wenn die Belastungsimpedanz (Z) kleiner ist als der voreingestellte Wert (Zu), und daß die Auswerteeinrichtung (6) einen voreingestellten niedrigen Wert (B2) als 1. Sollwert (b) durchschaltet, solange das Ausgangssignal (g) der Anzeigevorrichtung zur Bestimmung der Belastungsimpedanz (Z) sich in dem Schaltzustand befindet, der der Bedingung Z > Zu zugeordnet ist.

10. Anordnung nach einem der Ansprüche 1 bis 9, **dadurch gekennzeichnet**, daß die in der Auswerteeinrichtung (6) enthaltenen Schaltfunktion zumindest teilweise durch digitale arbeitende Bausteine realisiert sind.

11. Anordnung nach einem der Ansprüche 1 bis 10, **dadurch gekennzeichnet**, daß die in der Auswerteeinrichtung (6) enthaltenen Schaltfunktionen zumindest teilweise durch Programm-Algorithmen eines Mikroprozessors realisiert sind.

## Claims

1. An arrangement for cutting biological tissue with high-frequency current (1) having

   - a HF generator component (2) which is connected to a surgical probe (7) in such a manner that an electric arc (9) is generated between said surgical probe and the tissue to be cut (8),
   - an adjustment device (3) the output signal of which is transmitted to said HF generator in such a manner that the amplitude of the HF voltage applied to said tissue respectively of the HF current respectively of the HF power can be adjusted,
   **characterized by** the following features:
   - said adjustment device (3) adjusts the output voltage, the output current respectively the output power to a first desired value (b),
   - an indicator device (4) is providecd, the output signal of which is the measure of the size and intensity of said generated electric arc,
   - an evaluation device is provided to which said output signal of said indicator device and a second desired value for the size and intensity of said electric arc are transmitted, and which forms said first desired value (b) for said adjustment device as follows:
   if an output signal of said indicator device indicates too great an electric arc, the first desired value is decreased by at least one magnitude faster than it is increased if said output signal of said indicator device has the same amount of deviation toward too small values.

2. An arrangement according to claim 1, **characterized by** said evaluation unit (6) containing a subtraction circuit (12) for forming said first desired value for said adjustment device (b), said subtraction circuit (12) forming a difference signal (e) from said output signal of said indicator device (d) and from said second desired value for the size and intensity of said electric arc (c), as well an averaging circuit (13), which averages said difference signal (e) temporally.

3. An arrangement according to claim 1, **characterized by** said evaluation unit containing a subtraction circuit (12) for forming said first desired value for said adjustment device, said subtraction circuit (12) forming a difference signal (e) from said output signal of the indicator device (d) and from said second desired value for the size and intensity of said electric arc (c), as well as an integration circuit (14), which integrates said difference signal (e) temporally.

4. An arrangement according to one of the claims 1 to 3, **characterized by** said evaluation unit containing a limiting circuit (16) which does not permit said desired value to rise above a preset limit value.

5. An arrangement according to claim 4, **characterized by** said evaluation unit containing a limiting circuit so that said first desired value (b) is not under a preset limit value and not above a preset second limit value.

6. An arrangement according to one of the claims 1 to 4, **characterized by** said evaluation unit having a circuit for detecting an electric arc (18) and that as long as a preset value (B1) of an direct current signal (f) as said first desired value is applied to the output as long as said output signal of said indicator device (d) indicates that there is no electric arc and that said first desired value (b) is not formed by the comparison of said output signal from said indicator device (d) with said second desired value (c) until an electric arc occurs.

7. An arrangement according to one of the claims 1 to 6, **characterized by** in addition an indicator device (20) being provided which indicates the momentary impedance (Z) which is the load on said arrangement for cutting biological tissue at output (A1, A1') due to the tissue to be cut and that its output signal (g) is also transmitted to said evaluation unit (6).

8. An arrangement according to claim 7, **characterized by** said evaluation unit (6) setting a preset, low value (B2) as said desired value (b) as long as it recognizes from said output signal (g) of said indicator device for determining the momentary impedance that said impedance (Z) is larger than a preselected threshold value (Zu).

**9.** An arrangement according to claim 7,
**characterized by** said indicator device for determining the momentary impedance (20) being designed in such a manner that its output signal (g) only has two switching states, of which the one switching state occurs if said load impedance (Z) at said output (A1, A1') of said arrangement for cutting biological tissue is larger than a preset value (Zu) and the second switching state sets in if said load impedance (Z) is smaller than said preset value (Zu) and that said evaluation unit (6) switches through a preset low value (B2) as said first desired value (b) as long as said output signal (g) of said indicator device for determining said load impedance (Z) is in the switching state assigned to the condition Z > Zu.

**10.** An arrangement according to one of the claims 1 to 9,
**characterized by** the switching functions contained in said evaluation unit (6) being realized at least partially by digitally operating components.

**11.** An arrangement according to one of the claims 1 to 10,
**characterized by** the switching functions contained in said evaluation unit (6) being realized at least partially by program algorithms of a microprocessor.

**Revendications**

**1.** Dispositif pour inciser un tissu biologique à l'aide d'un courant à haute fréquence (1), comportant :

- une partie formant générateur HF (2), qui est reliée à une sonde chirurgicale (7) de telle sorte qu'un arc électrique (9) est produit entre la sonde chirurgicale et le tissu à inciser (8),
- un dispositif de régulation (3), dont le signal de sortie est appliqué à la partie formant générateur HF, de sorte que l'amplitude de la tension HF appliquée au tissu ou du courant HF ou de la puissance HF est réglée,
caractérisé par les caractéristiques suivantes :
- le dispositif de régulation (3) règle la tension de sortie, le courant de sortie ou la puissance de sortie sur une première valeur de consigne (b),
- il est prévu un dispositif d'affichage (4), dont le signal de sortie est une mesure de l'intensité de l'arc électrique produit,
- il est prévu un dispositif d'évaluation, auquel sont appliqués le signal de sortie du dispositif d'affichage et une seconde valeur de consigne de la grandeur de l'arc électrique, et qui forme la première valeur de consigne (b) pour le dispositif de régulation comme suit :
dans le cas d'un signal de sortie du dispositif d'affichage qui affiche une valeur trop importante de l'arc électrique, la première valeur de consigne est réduite d'au moins un ordre de grandeur plus rapidement qu'elle n'est augmentée, lorsque le signal de sortie du dispositif d'affichage présente la même valeur absolue de l'écart vers des valeurs trop faibles.

**2.** Dispositif selon la revendication 1, caractérisé en ce que l'unité d'exploitation (6) contient, pour la formation de la 1-ère valeur de consigne pour le dispositif de régulation (b), un circuit soustracteur (12), qui forme un signal de différence (e) à partir du signal de sortie du dispositif d'affichage (d) et de la 2-ème valeur de consigne pour la valeur de l'arc électrique (c), ainsi qu'un circuit (13) de formation de la valeur moyenne, qui forme la moyenne dans le temps de ce signal de différence (e).

**3.** Dispositif selon la revendication 1, caractérisé en ce que l'unité d'exploitation pour la formation de la 1-ère valeur de consigne pour le dispositif de régulation contient un circuit soustracteur (12), qui forme un signal de différence (e) à partir du signal de sortie du dispositif d'affichage (d) et de la 2-ème valeur de consigne pour la valeur de l'arc électrique (c), ainsi qu'un circuit d'intégration (14), qui intègre dans le temps ce signal de différence (e).

**4.** Dispositif selon l'une des revendications 1 à 3, caractérisé en ce que le dispositif d'évaluation contient un circuit limiteur (16), qui ne permet pas à la valeur de consigne d'augmenter au-delà d'une valeur limite préréglée.

**5.** Dispositif selon la revendication 4, caractérisé en ce que le dispositif d'exploitation contient un circuit limiteur de telle sorte que la 1-ère valeur de consigne (b) n'est pas inférieure à une valeur limite préréglée et n'est pas supérieure à une 2-ème valeur limite préréglée.

**6.** Dispositif selon l'une des revendications 1 à 4, caractérisé en ce que le dispositif d'exploitation possède un circuit servant à identifier (18) un arc électrique et que, tant qu'une valeur préréglée (B1) d'un signal continu (f) est appliquée à l'entrée en tant que 1-ère valeur de consigne, le signal de sortie du dispositif d'affichage (d) indique qu'aucun arc électrique n'est présent et que c'est seulement lors de l'apparition d'un arc électrique que la 1-ère valeur de consigne (b) est formée à partir de la comparaison entre le signal de sortie du dispositif d'affichage (d) et la 2-ème valeur de consigne (c).

**7.** Dispositif selon l'une des revendications 1 à 6, caractérisé en ce qu'il est prévu en supplément un dispositif d'affichage (20), qui affiche l'impédance instantanée (Z) avec laquelle le dispositif servant à inciser un tissu biologique est chargé par le tissu à inciser, à la sortie (A1, A1') et que son signal de sortie (g) est également envoyé au dispositif d'évaluation (6).

8. Dispositif selon la revendication 7, caractérisé en ce que le dispositif d'évaluation (6) règle une faible valeur préréglée (B2) en tant que valeur de consigne (b), tant qu'il identifie à partir du signal de sortie (g) du dispositif d'affichage pour déterminer l'impédance instantanée le fait que l'impédance (Z) est supérieure à une valeur de seuil présélectionnée (Zu).

9. Dispositif selon la revendication 7, caractérisé en ce que le dispositif d'affichage pour déterminer l'impédance instantanée (20) est agencé de telle sorte que son signal de sortie (g) possède seulement deux états de commutation, dont l'un apparaît lorsque l'impédance de charge (Z) à la sortie (1,1') du dispositif pour inciser un tissu biologique est supérieure à une valeur préréglée (Zu), tandis que le second état de commutation est présent lorsque l'impédance de charge (Z) est inférieure à la valeur préréglée (Zu) et que le dispositif d'exploitation (6) transmet une faible valeur préréglée (B2) en tant que 1-ère valeur de consigne (b), tant que le signal de sortie (g) du dispositif d'affichage servant à déterminer l'impédance de charge (Z) est situé dans l'état de commutation, qui est associé à la condition Z > Zu.

10. Dispositif selon l'une des revendications 1 à 9, caractérisé en ce que la fonction de commutation contenue dans le dispositif d'exploitation (6) est réalisée au moins partiellement par des modules travaillant numériquement.

11. Dispositif selon l'une des revendications 1 à 10, caractérisé en ce que les fonctions de commutation contenues dans le dispositif d'exploitation (6) sont réalisées au moins partiellement par des algorithmes de programme d'un microprocesseur.

**Fig. 1**

Fig. 2

Fig. 3

Fig. 4

Fig. 5

Fig. 6

Fig. 7

**Fig. 8**

EP 0 598 805 B1

Fig. 9